(19) Europäisches Patentamt
European Patent Office
Office européen des brevets

(11) Numéro de publication: **0 067 080**
**B1**

(12) **FASCICULE DE BREVET EUROPEEN**

(45) Date de publication du fascicule du brevet: **21.11.84**

(21) Numéro de dépôt: **82400804.9**

(22) Date de dépôt: **03.05.82**

(51) Int. Cl.³: **C 07 C 103/375**, C 07 C 103/38, C 07 C 102/00, C 07 D 223/10, C 07 C 103/76, C 07 C 103/78

(54) **Nouveaux amides N-benzylfluorés et leur procédé de préparation.**

(30) Priorité: **15.05.81 FR 8109696**

(43) Date de publication de la demande: **15.12.82 Bulletin 82/50**

(45) Mention de la délivrance du brevet: **21.11.84 Bulletin 84/47**

(84) Etats contractants désignés: **AT BE CH DE FR GB IT LI LU NL SE**

(56) Documents cités:
**FR - A - 2 111 766**

**JOURNAL OF THE AMERICAN CHEMICAL SOCIETY, vol. 80, 20 aout 1958, pages 4317-4319, Washington D.C., USA H.E. ZAUGG et al.: "The amidomethylation reaction. Preparation of m- and p-aminomethylphenylacetic acids"**
**EUGEN MULLER: "Methoden der organischen Chemie", Houben Weyl, 4ieme edition, vol. XI/1, 1957, pages 795-805, Georg Thieme Verlag, Stuttgart, DE.**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526 Chemin du Bois, F-69140 Rillieux (FR)**
Inventeur: **Reppelin, Michel, 10 Chemin Neuf, F-69660 Collonges-au-Mont d'Or (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

EP 0 067 080 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux amides N-benzylfluorés; elle concerne également un procédé de préparation de ces composés.

Les amides N-benzylfluorés selon l'invention ont pour formule générale:

$$(X)_n-Ar-CH_2-NR_1-COR_2 \quad (I)$$

dans laquelle:

X représente un radical choisi parmi le groupe comprenant F, $CF_3$, $OCF_3$ et $SCF_3$,

Ar représente le radical phénylène ou un radical phénylène substitué par un radical choisi parmi le groupe comprenant les radicaux Cl, Br, CN, $NO_2$, $NH_2$, CHO, COOH, COR, COOR où R est un radical alkyle ayant de 1 à 6 atomes de carbone, alkyle ou alkoxy ayant de 1 à 6 atomes de carbone, phényle ou phénoxy,

$R_1$ et $R_2$ identiques ou différents représentent un hydrogène, ou un radical alkyle ayant de 1 à 6 atomes de carbone environ, un radical phényle $R_1$ et $R_2$ pouvant être liés pour former un cycle,

n est égal à 1, 2 ou 3.

Un objet particulier de l'invention sont les composés de formule I dans laquelle n = 1.

Un autre objet particulier de la présente invention sont les composés de formule I dans laquelle $R_1$ et $R_2$ représentent simultanément un atome d'hydrogène.

Encore plus particulièrement, parmi ces derniers, l'invention concerne le N-métatrifluorométhylbenzylformamide de formule:

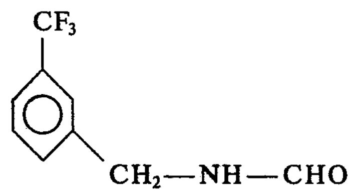

le N-ortho et le N-parafluorobenzylformamide de formules:

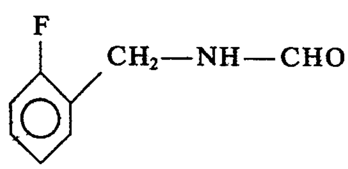

et

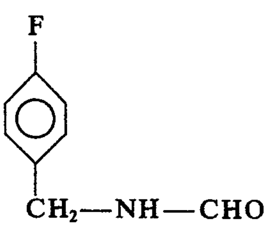

le N-ortho et le N-paratrifluorométhoxybenzylformamide de formules:

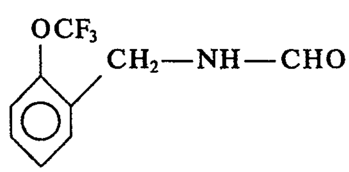

et

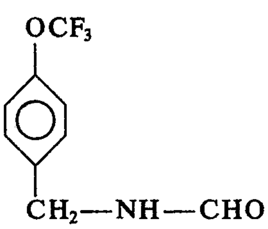

et le N-ortho et le N-paratrifluoromethylthiobenzylformamide de formules:

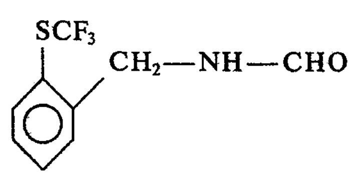

et

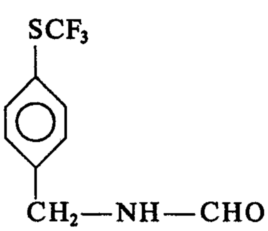

Un autre objet particulier de la présente invention sont les composés de formule I dans laquelle $R_1$ représente un atome d'hydrogène et $R_2$ représente un radical méthyle.

Encore plus particulièrement, parmi ces derniers, l'invention concerne le N-métatrifluorométhylbenzylacétamide de formule:

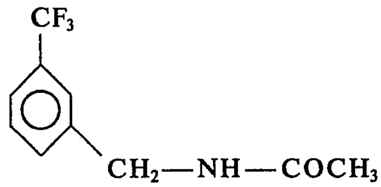

le N-ortho et le N-parafluorobenzylacétamide de formules:

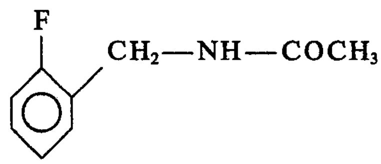

et

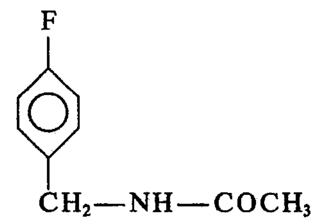

le N-ortho et le N-paratrifluorométhoxybenzylacétamide de formules:

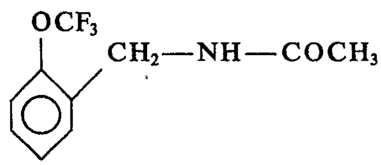

et

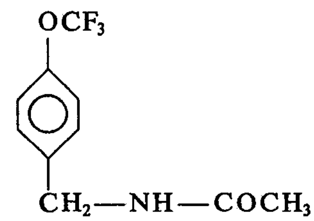

le N-ortho et le N-paratrifluorométhylthiobenzylacétamide de formules:

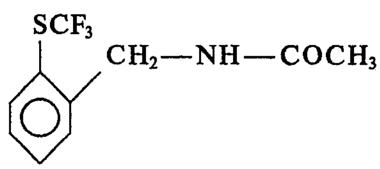

et

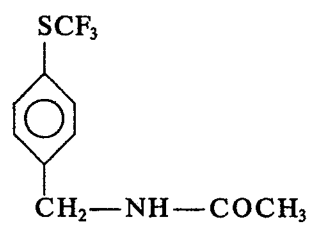

On peut citer comme autres exemples non limitatifs de composés I selon la présente invention:

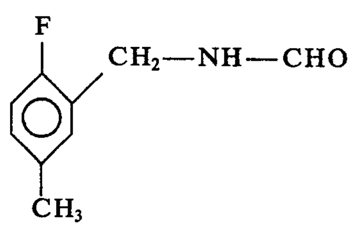

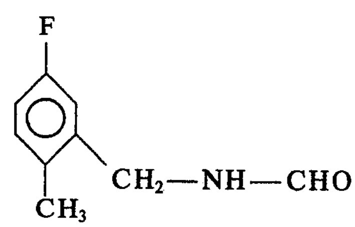

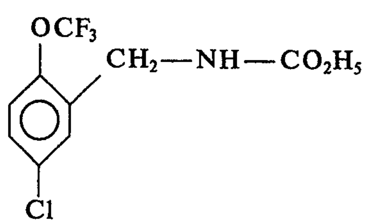

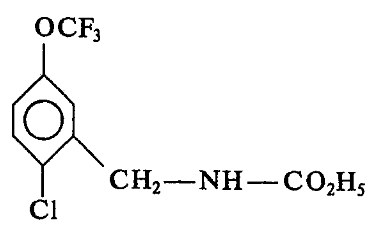

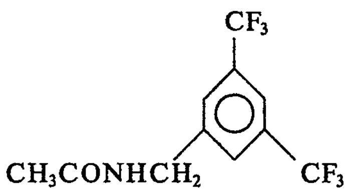

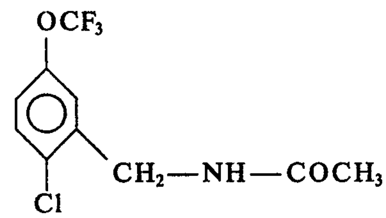

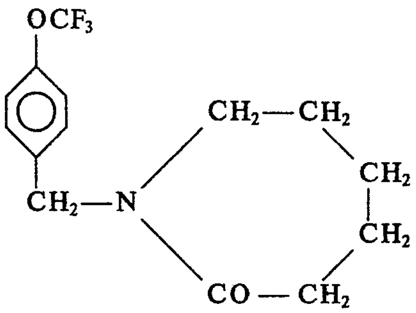

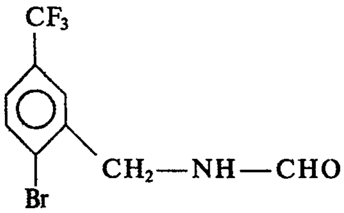

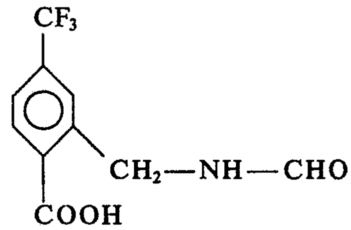

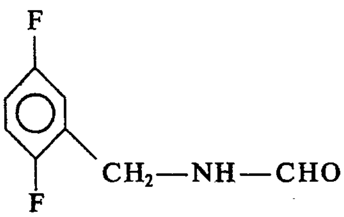

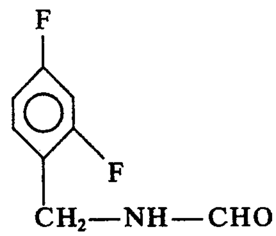

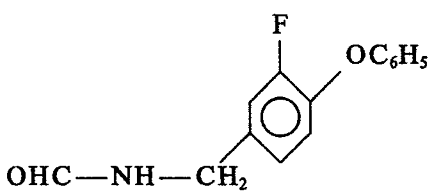

L'invention concerne également un procédé de préparation des composés de formule I selon lequel on fait réagir, en présence d'acide fluorhydrique, un composé de formule:

$(X)_n-ArH$ (II)

où X, n et Ar ont la signification précédente avec un composé de formule:

$OH-CH_2-NR_1-COR_2$ (III)

dans laquelle $R_1$ et $R_2$ ont la signification précédente.

On connaissait dans l'art antérieur (JACS Vol. 80 p.4317—4319) la réaction de l'acide phénylacétique en solution acide sulfurique concentrée avec le N-hydroxychloroacétamide qui sont tous deux des composés comportant un substituant activant, respectivement $CH_2COOH$ et $CH_2Cl$.

L'invention apporte la possibilité de faire réagir dans l'HF des composés comportant des substituants désactivants.

Selon l'invention, l'acide fluorhydrique mis en œuvre est, de préférence, de l'acide fluorhydrique anhydre. On peut également utiliser de l'acide fluorhydrique aqueux dont la concentration est, de préférence, supérieure à 90%.

Selon un mode particulier de mise en œuvre de l'invention, on utilise un composé de formule (II) dans laquelle $n = 1$.

Selon un mode particulier de mise en œuvre de l'invention, on utilise un composé de formule (III) dans laquelle $R_1$ et $R_2$ sont simultanément un atome d'hydrogène.

Selon un autre mode particulier de mise en œuvre de l'invention, on utilise un composé de formule

(III) dans laquelle $R_1$ représente un atome d'hydrogène et $R_2$ représente un radical méthyle.

L'invention est donc particulièrement bien adaptée à la mise en réaction du N-hydroxyméthylformamide $OHCH_2NHCHO$ ou du N-hydroxyméthylacétamide $OHCH_2NHCOCH_3$ avec le trifluorométhylbenzène, le fluorobenzène, le trifluorométhoxybenzène et le trifluorométhylthiobenzène.

Selon un mode de réalisation préférentiel du procédé selon l'invention, on utilise une quantité d'acide fluorhydrique telle que le rapport molaire de l'acide fluorhydrique au composé II est compris entre 5 et 50. Encore plus préférentiellement ce rapport est compris entre 20 et 40.

Selon un autre mode de réalisation préférentiel du procédé selon l'invention, on utilise les composés II et III en quantités telles que le rapport molaire du composé II au composé III est compris entre 0,5 et 2. Encore plus préférentiellement, ce rapport est compris entre 0,8 et 1,2.

Pour une bonne mise en œuvre du procédé selon l'invention, on opère à une température généralement comprise entre 0 et 100° C. La température sera plus finement choisie en fonction du composé II lorsque le composé comportera un substituant plus au moins désactivant, la température sera plus ou moins élévée.

Lorsque la température sera supérieure à la température d'ébullition de l'HF (20° C), on opérera dans un réacteur fermé sous pression autogène. Dans le cas contraire, on préfèrera opérer sous pression atmosphérique bien que des pressions supérieures ou inférieures à la pression atmosphérique ne soient pas exclues du domaine de l'invention.

Les temps de réaction sont généralement compris entre quelques minutes et 20 H 00.

La séparation des produits de la réaction peut se faire par toute méthode connue de l'homme de l'art et, en particulier, par coulée du mélange réactionnel brut sur de la glace et extraction à l'aide de solvants organiques ou par distillation de l'HF (distillation flash par exemple).

Les composés I ont un réel intérêt industriel dans la mesure où ils peuvent permettre l'accès, soit aux phénylacétonitriles de formule:

$$(X)_n-Ar-CH_2-CN \qquad (IV)$$

soit aux benzylamines de formule:

$$(X)_n-Ar-CH_2-NH_2 \qquad (V)$$

soit encore aux benzylamines N-substitués de formule:

$$(X)_n-Ar-CH_2-NR_1H \qquad (VI)$$

où Ar, X, n et $R_1$ ont la signification précédente. Ces composés IV, V et VI ont de très intéressants intermédiaires pour la synthèse de nombreux composés à activité phytosanitaire ou pharmaceutique.

Les composés V peuvent être obtenus par hydrolyse acide ou basique des composés de formule I dans laquelle $R_1 = H$ et $R_2$ est un radical alkyle ou un atome d'hydrogène.

Les composés VI résultent également de l'hydrolyse de composés de formule I dans laquelle $R_1$ est un radical alkyle et $R_2$ est un radical alkyle ou un atome d'hydrogène.

Les composés IV peuvent être obtenus à partir des composés de formule I dans laquelle $R_1$ et $R_2$ sont simultanément un atome d'hydrogène. Pour cela, on porte à une température entre 450° C et 550° C le composé I en présence d'un catalyseur constitué de silice ayant une surface spécifique comprise entre 200 et 300 $m^2/g$, un volume poreux total compris entre 1 et 1,5 $cm^3/g$, un diamètre moyen des pores compris entre 100 et 200 Å, un pH d'échange inférieur à 3, une teneur en fluor exprimée en F comprise entre 0,05 et 2% en poids par rapport à la silice et une teneur en sodium exprimée en $Na_2O$ inférieure à 0,01% environ en poids par rapport à la silice et une teneur en sodium exprimée en $Na_2O$ inférieure à 0,01% environ en poids par rapport à la silice.

Cette silice, qui fait l'objet d'une demande de brevet au nom de la demanderesse, est préparée en ajoutant à une solution aqueuse d'acide fluorhydrique, une solution aqueuse de silicate de soude à une température comprise entre −5° C et 15° C tout en maintenant la teneur en $SiO_2$ inférieure à 15% en poids du mélange réactionnel jusqu'à ce que le pH du milieu réactionnel atteigne une valeur comprise entre 3 et 4,5 en laissant gélifier le mélange, en concassant en grains l'hydrogel obtenu et en lavant les grains obtenus pour éliminer les sels fluorés solubles et en séchant les grains lavés à une température comprise entre 150° C et 600° C.

On met en œuvre de préférence du silicate de sodium contenant du $SiO_2$ et du $Na_2O$ dans un rapport molaire égal à environ 3 et on met en œuvre entre 1 et 1,5 mole de HF par mole de $SiO_2$.

L'invention va être maintenant plus complètement décrite, à l'aide des exemples qui vont suivre. Ces exemples ne sauraient être considérés comme limitant de façon quelconque l'invention.

Exemple 1

Préparation de N-métatrifluorométhylbenzylformamide

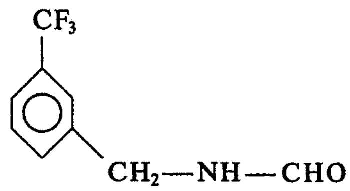

Dans un réacteur en acier inoxydable muni d'un agitateur, on introduit 100 g (5 moles) d'HF anhydre qu'on refroidit à 0° C environ. On introduit ensuite lentement 9 g (0,12 mole) d'hydroxyméthylformamide $OHCH_2NHCHO$ en maintenant la température entre 0 et 5° C environ. On introduit ensuite 17 g (0,116 mole) de trifluorométhylbenzène. On ferme le réacteur et on porte pendant 22 heures à 50° C sous agitation. On refroidit le réacteur à 0—5° C. On vers le mélange réactionnel sur 200 g de glace et on extrait les produits organiques au chlorure de méthylène (5 × 100 $cm^3$). La phase organique recueillie est ensuite lavée à l'eau déminéralisée jusqu'à obtention d'une eau de lavage ayant un pH compris entre 5 et 6. La phase organique est séchée puis évaporée sous pression réduite (15—20 mm de Hg).

On récupère ainsi 23,4 g (0,115 mole) d'un composé dont l'analyse par chromatographie en phase vapeur, infrarouge et spectrométrie de masse révèle qu'il s'agit de N-métatrifluorométhylbenzylformamide.

Exemple 2

Préparation de N-métatrifluorométhylbenzylacétamide

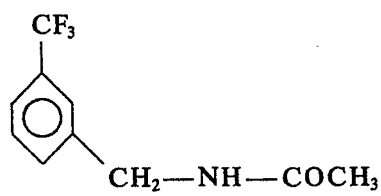

On opère comme dans l'exemple 1 mais en utilisant 6,9 g (0,06 mole) de N-hydroxyméthylacétamide $OH-CH_2-NH-COCH_3$ et 8,5 g (0,058 mole) de trifluorométhylbenzène.

On effectue la réaction à 50° C pendant 13 heures.

On récupère 11 g (0,05 mole) d'un composé dont l'analyse révèle qu'il s'agit du N-métatrifluorométhylbenzylacétamide.

Exemple 3

Préparation des N-orthotrifluorométhoxybenzylformamide
et N-paratrifluorométhoxybenzylformamide

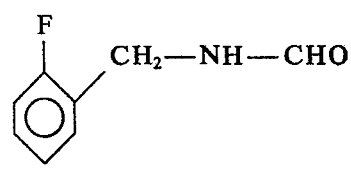

et

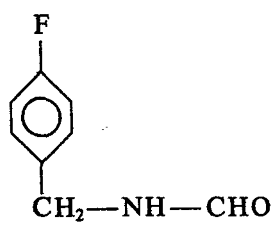

On opère comme dans l'exemple 1 mais en utilisant 32,4 g (0,2 mole) de trifluorométhoxybenzène et 22,5 g (0,3 mole) d'hydroxyméthylformamide.

On effectue la réaction à 13° C pendant 3 heures.

On récupère 37,2 g d'un mélange dont l'analyse par chromatographie en phase vapeur montre qu'il contient 15% de N-orthotrifluorométhoxybenzylformamide et 85% de N-paratrifluorométhoxybenzylformamide que l'on sépare par tout procédé connu de l'homme de l'art comme par distillation par exemple.

## Exemple 4

Préparation des N-ortho et N parafluorobenzylformamides

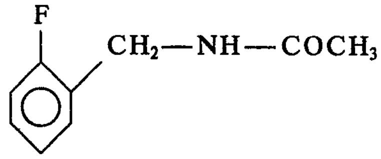

et

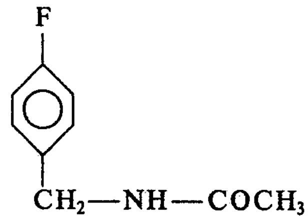

On opère comme dans l'exemple 1 mais en utilisant 50 g (2,5 moles) d'HF, 19,2 g (0,2 mole) de fluorobenzène et 20 g (0,26 mole) d'hydroxyméthylformamide.

La réaction a lieu à 20° C pendant 15 minutes.

On obtient 26 g d'un mélange liquide dont l'analyse par chromatographie en phase vapeur révèle qu'il contient 10% d'orthofluorobenzylformamide et 90% de parafluorobenzylformamide.

## Exemple 5

Préparation des N-ortho et N-parafluorobenzylacétamides

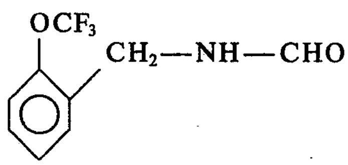

et

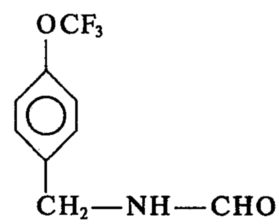

On opère comme dans l'exemple 1 en utilisant 50 g (2,5 moles) d'HF, 9,6 g (0,1 mole) de fluorobenzène et 10 g (0,11 mole) d'hydroxyméthylacétamide.

La réaction est effectuée à 20° C pendant 20 heures.

On obtient 12 g d'un mélange contenant 5% d'orthofluorobenzylacétamide et 95% de parafluorobenzylacétamide.

## Exemple 6

Préparation des N-ortho et N-para fluorobenzyl N-méthylacétamides

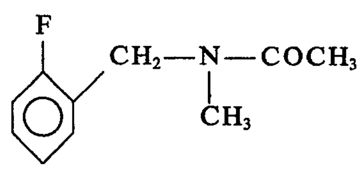

et

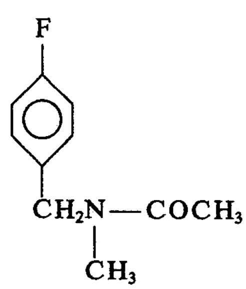

On opère comme dans l'exemple 1 avec 100 g (5 moles) d'HF, 19,2 g (0,2 mole) de fluorobenzène et 36 g (0,35 mole) de N-hydroxyméthyl N-méthylacétamide.

La réaction est conduite à 20° C pendant 15 minutes.

On obtient 36 g d'un mélange contenant 25% de N-orthofluorobenzyl N-méthylacétamide et 75% de N-parafluorobenzyl N-méthylacétamide.

Exemple 7

Préparation des N-fluoro-2 méthyl-5 benzylformamide
et N-fluoro-3 méthyl-6 benzylformamide

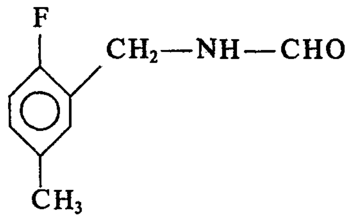

et

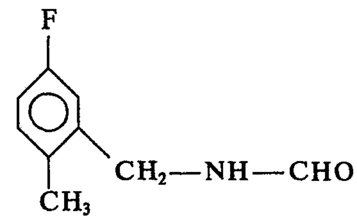

On opère comme dans l'exemple 1 avec 100 g d'HF (5 moles), 25,3 g (0,23 mole) de parafluorotoluène et 30 g (0,4 mole) de N-hydroxyméthylformamide.

La réaction est conduite è 20° C pendant 20 minutes.

On récupère 36,3 g d'un mélange contenant 40% de N-fluoro-2 méthyl-5 benzylformamide et 60% de N-fluoro-3 méthyl-6 benzylformamide.

Exemple 8

Préparation des N-ortho et N-paratrifluorométhylthiobenzylformamide

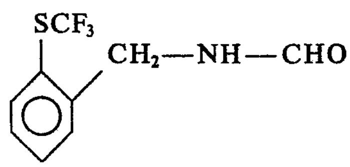

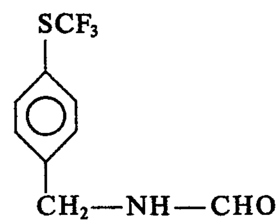

On opère comme dans l'exemple 1 mais en utilisant 17,8 g (0,1 mole) de trifluorométhylthiobenzène et 7,5 g (0,1 mole) d'hydrométhylformamide.

On effectue la réaction à 10° C pendant 3 heures 50 minutes.

On récupère 21,2 g d'un mélange dont l'analyse par chromatographie en phase vapeur montre qu'il contient environ 18% de N-orthotrifluorométhylthiobenzylformamide et environ 82% de N-paratrifluorométhylthiobenzylformamide.

Exemple 9

Préparation du N-difluoro-2,4 benzylformamide

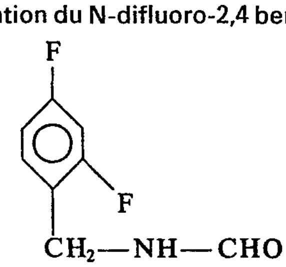

On opère comme dans l'exemple 1 mais en utilisant 22,8 g (0,2 mole) de métadifluorobenzène, 22,5 g (0,3 mole) d'hydroxyméthylformamide.

On effectue la réaction à 50° C pendant 4 heures. On récupère 35,1 g d'un composé dont les analyses par chromatographies en phase vapeur infrarouge et spectrométrie de masse révèlent qu'il s'agit de N-difluoro-2,4 benzylformamide.

Exemple 10

Préparation du N-métatrifluorométhylbenzylbenzamide

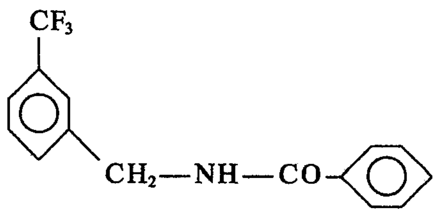

On opère comme dans l'exemple 1 mais en utilisant 14,6 g (0,1 mole) de trifluorométhylbenzène et 15,1 g (0,1 mole) de N-hydroxyméthylbenzamide.

On effectue la réaction à 20° C pendant 23 heures.

On récupère 16,4 g d'un composé dont les analyses par chromatographies en phase vapeur infrarouge et spectrométrie de masse révèlent qu'il s'agit de N-métatrifluorométhylbenzylbenzamide.

Exemple 11

Préparation du N-paratrifluorométhoxybenzyl ε-caprolactame

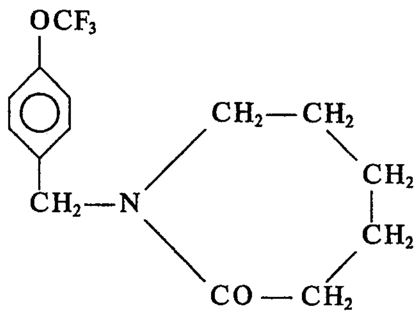

On opère comme dans l'exemple 1 mais en utilisant 32,4 g (0,2 mole) de trifluorométhoxybenzène et 42,9 g (0,3 mole) de N-hydroxyméthyl ε-caprolactame.

On effectue la réaction à 10° C pendant 22 heures.

On récupère 55,2 g d'un composé dont les analyses par chromatographies en phase vapeur, infrarouge et spectrométrie de masse révèlent qu'il s'agit de N-paratrifluorométhoxybenzyl ε-caprolactame.

**Revendications pour les etats contractants: BE, CH, DE, FR, GB, IT, LI, LU, NL, SE**

1. Amides N-benzylfluorés de formule (I)

$$(X)_n-Ar-CH_2-NR_1-COR_2 \quad (I)$$

dans laquelle:
X représente un radical choisi parmi le groupe comprenant F, $CF_3$, $OCF_3$ et $SCF_3$,
Ar représente le radical phénylène ou un radical phénylène substitué par un radical choisi parmi le groupe comprenant: Cl, Br, CN, $NO_2$, $NH_2$, CHO, COOH, COR, COOR où R est un radical alkyle ayant de 1 à 6 atomes de carbone, les radicaux alkyle et alkoxy ayant de 1 à 6 atomes de carbone, le radical phényle et le radical phénoxy,
$R_1$ et $R_2$ identiques ou différents représentent chacun un hydrogène ou un radical alkyle ayant de 1 à 6 atomes de carbone un radical phényle, $R_1$ et $R_2$ pouvant être liés pour former un cycle,
et n est égal à 1, 2 ou 3.

2. Amides selon la revendication 1 caractérisés en ce que Ar représente le radical phénylène.

3. Amides selon l'une quelconque des revendications précédentes caractérisés en ce que n = 1.

4. Amides selon l'une quelconque des revendications précédentes caractérisés en ce que $R_1$ et $R_2$ représentent simultanément un atome d'hydrogène.

5. Amides selon les revendications 1, 2, 3 et 4 caractérisés en ce qu'ils ont pour formule:

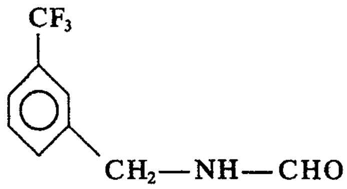

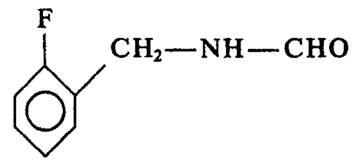

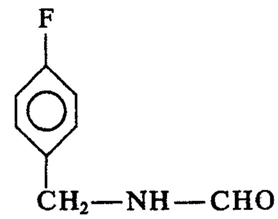

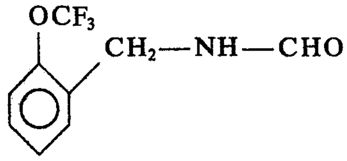

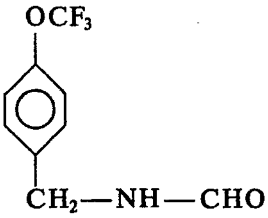

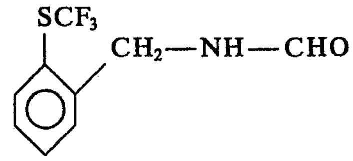

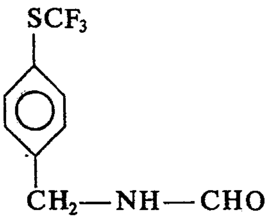

6. Amides selon l'une quelconque des revendications 1 à 3 caractérisés en ce que $R_1$ représente un atome d'hydrogène et $R_2$ représente un radical méthyle.

7. Amides selon les revendications 1, 2, 3 et 6 caractérisés en ce qu'ils ont pour formula:

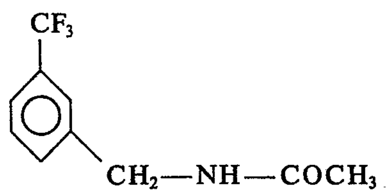

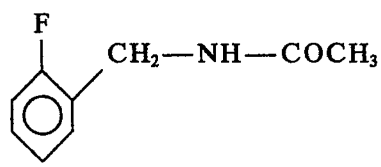

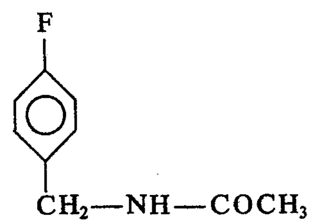

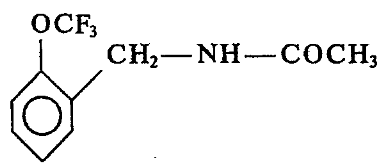

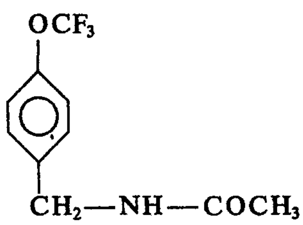

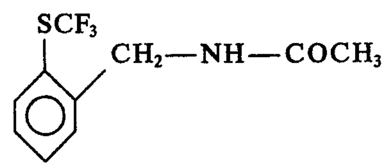

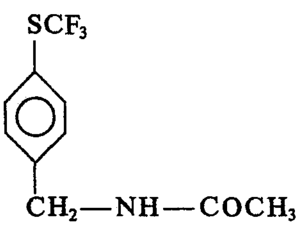

8. Procédé de préparation d'un amide selon l'une quelconque des revendications précédentes caractérisé en ce qu'on fait réagir, en présence d'acide fluorhydrique, un composé de formule (II)

$$(X)_n-ArH \qquad (II)$$

avec un composé de formule (III)

$$OH-CH_2-NR_1-COR_2 \qquad (III)$$

dans lesquelles X, Ar, $R_1$, $R_2$ ont les mêmes signification que dans la revendication 1.

9. Procédé selon la revendication 8 caractérisé en ce que l'acide fluorhydrique est de l'acide fluorhydrique anhydre.

10. Procédé selon l'une quelconque des revendications 8 et 9 caractérisé en ce que Ar représente le radical phénylène.

11. Procédé selon l'une quelconque des revendications 8 à 10 caractérisé en ce que $n = 1$.

12. Procédé selon l'une quelconque des revendications 8 à 11 caractérisé en ce que dans la formule (III), $R_1$ et $R_2$ sont simultanément un atome d'hydrogène.

13. Procédé selon l'une quelconque des revendications 8 à 11 caractérisé en ce que dans la formule (III), $R_1$ représente un atome d'hydrogène et $R_2$ un radical méthyle.

14. Procédé selon l'une quelconque des revendications 8 à 13 caractérisé en ce que l'on met en œuvre une quantité d'acide fluorhydrique telle que le rapport molaire de l'acide fluorhydrique au composé de formule II est compris entre 5 et 50.

15. Procédé selon l'une quelconque des revendications 8 à 14 caractérisé en ce que le rapport molaire du composé II au composé III est compris entre 0,5 et 2.

16. Procédé selon l'une quelconque des revendications 8 à 15 caractérisé en ce que l'on opère à une température comprise entre 0° C et 100° C.

**Revendications pour l'etat contractant: AT**

1. Procédé de préparation d'un amide caractérisé en ce qu'on fait réagir, en présence d'acide fluorhydrique, un composé de formule (II)

$$(X)_n{-}ArH \qquad (II)$$

dans laquelle X représente un radical choisi parmi le groupe comprenant F, $CF_3$, $OCF_3$ et $SCF_3$, n est égal à 1, 2 ou 3, Ar représente un radical phénylène ou phénylène substitué par un radical choisi parmi le groupe comprenant Cl, Br, CN, $NO_2$, $NH_2$, CHO, COOH, COR, COOR où R est un radical alkyle ayant de 1 à 6 atomes de carbone, les radicaux alkyle et alkoxy ayant de 1 à 6 atomes de carbone, le radical phényle et le radical phénoxy; avec un composé de formule (III)

$$OH{-}CH_2{-}NR_1{-}COR_2$$

dans laquelle $R_1$ et $R_2$ identiques ou différents représentent un hydrogène ou un radical alkyle ayant de 1 à 6 atomes de carbone, un radical phényle $R_1$ et $R_2$ pouvant être liés pour former un cycle.

2. Procédé selon la revendication 1 caractérisé en ce que l'acide fluorhydrique est de l'acide fluorhydrique anhydre.

3. Procédé selon la revendication 1 caractérisé en ce que Ar représente le radical phénylène.

4. Procédé selon l'une quelconque des revendications 1 à 3 caractérisé en ce que $n = 1$.

5. Procédé selon l'une quelconque des revendications 1 à 4 caractérisé en ce que dans la formule (III), $R_1$ et $R_2$ sont simultanément un atome d'hydrogène.

6. Procédé selon l'une quelconque des revendications 1 à 4 caractérisé en ce que dans la formule (III), $R_1$ représente un atome d'hydrogène et $R_2$ un radical méthyle.

7. Procédé selon l'une quelconque des revendications 1 à 6 caractérisé en ce que l'on met en œuvre une quantité d'acide fluorhydrique telle que le rapport molaire de l'acide fluorhydrique au composé de formule II est compris entre 5 et 50.

8. Procédé selon l'une quelconque des revendications 1 à 7 caractérisé en ce que le rapport molaire du composé II au composé III est compris entre 0,5 et 2.

9. Procédé selon l'une quelconque des revendications 1 à 8 caractérisé en ce que l'on opère à une température comprise entre 0° C et 100° C.

**Patentansprüche für die Vertragsstaaten: BE, CH, DE, FR, GB, IT, LI, LU, NL, SE**

1. N-Fluorbenzylamide der Formel:

$$(X)_n{-}Ar{-}CH_2{-}NR_1{-}COR_2 \qquad (I)$$

in der
X einen Rest aus der Gruppe von F, $CF_3$, $OCF_3$ und $SCF_3$ bedeutet,
Ar den Phenylenrest oder einen mit einem Rest aus der Gruppe von Cl, Br, CN, $NO_2$, CHO, COOH, COR, COOR, wobei R ein Alkylrest mit 1 bis 6 Kohlenstoffatomen ist, den Alkyl- und Alkoxyresten mit 1 bis 6 Kohlenstoffatomen, den Phenyl- und Phenoxyresten substituierten Phenylenrest bedeutet,
$R_1$ und $R_2$, die gleich oder unterschiedlich sein können, jeweils ein Wasserstoffatom oder ein Alkylradikal mit 1 bis 6 Kohlenstoffatomen, ein Phenylrest sind, und wobei $R_1$ und $R_2$ miteinander zu einem Ring verbunden sein können, und
n gleich 1, 2 oder 3
ist.

2. Amide nach Anspruch 1, dadurch gekennzeichnet, daß Ar der Phenylenrest ist.

3. Amide nach einem der vorangehenden Ansprüche, dadurch gekennzeichnet, daß n gleich 1 ist.

4. Amide nach einem der vorangehenden Ansprüche, dadurch gekennzeichnet, daß $R_1$ und $R_2$ gleichzeitig ein Wasserstoffatom bedeuten.

5. Amide nach den Ansprüchen 1 bis 4, dadurch gekennzeichnet, daß sie die Formel besitzen

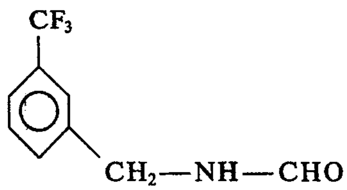
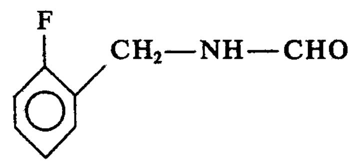
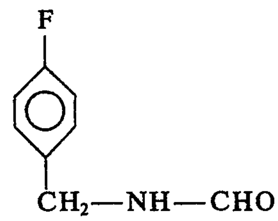
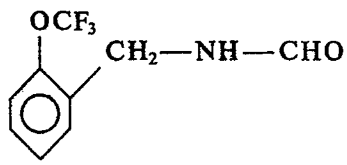
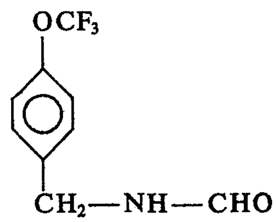
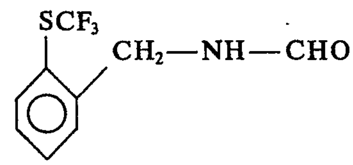
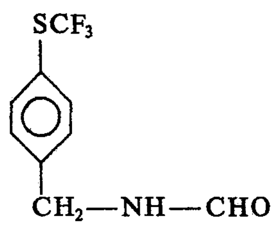

6. Amide nach einem der Ansprüche 1 bis 3, dadurch gekennzeichnet, daß $R_1$ ein Wasserstoffatom ist und $R_2$ den Methylrest bedeutet.

7. Amide nach den Ansprüchen 1, 2, 3 und 6, dadurch gekennzeichnet, daß sie die Formel haben:

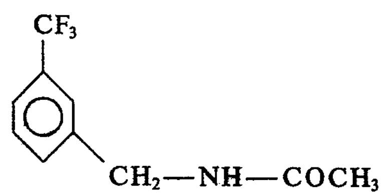
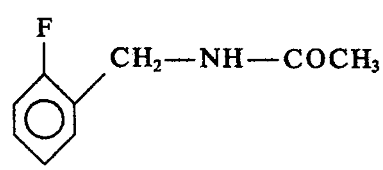
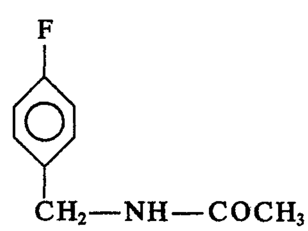
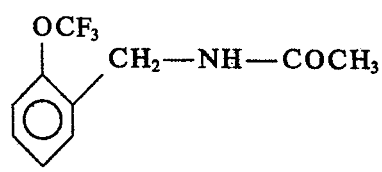
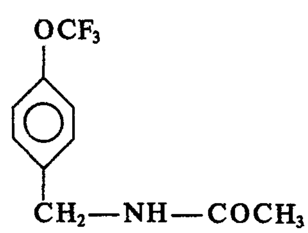
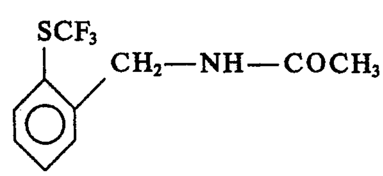
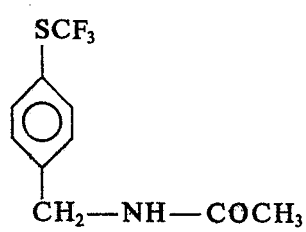

8. Verfahren zur Herstellung eines Amides nach einem der vorangehenden Ansprüche, dadurch gekennzeichnet, daß man eine Verbindung der Formel (II)

$$(X)_n—ArH \qquad (II)$$

mit einer Verbindung der Formel (III)

$$OH—CH_2—NR_1—COR_2 \qquad (III)$$

in denen X, Ar, $R_1$ und $R_2$ die in Anspruch 1 genannten Bedeutungen besitzen, in Gegenwart von Fluorwasserstoff umsetzt.

9. Verfahren nach Anspruch 8, dadurch gekennzeichnet, daß die Fluorwasserstoffsäure wasserfrei ist.

10. Verfahren nach einem der Ansprüche 8 und 9, dadurch gekennzeichnet, daß Ar den Phenylenrest bedeutet.

11. Verfahren nach einem der Ansprüche 8 bis 10, dadurch gekennzeichnet, daß n gleich 1 ist.

12. Verfahren nach einem der Ansprüche 8 bis 11, dadurch gekennzeichnet, daß in Formel (III) $R_1$ und $R_2$ gleichzeitig ein Wasserstoffatom bedeuten.

13. Verfahren nach einem der Ansprüche 8 bis 11, dadurch gekennzeichnet, daß in Formel (III) $R_1$ ein Wasserstoffatom und $R_2$ den Methylrest bedeutet.

14. Verfahren nach einem der Anspürche 8 bis 13, dadurch gekennzeichnet, daß eine derartige Menge Fluorwasserstoffsäure eingesetzt wird, daß das Molverhältnis zwischen der Fluorwasserstoffsäure und der Verbindung nach Formel (II) zwischen 5 und 50 liegt.

15. Verfahren nach einem der Ansprüche 8 bis 14, dadurch gekennzeichnet, daß daß das Molverhältnis der Verbindung (II) zur Verbindung (III) zwischen 0,5 und 2 liegt.

16. Verfahren nach einem der Ansprüche 8 bis 15, dadurch gekennzeichnet, daß man bei einer Temperatur zwischen 0 und 100° C arbeitet.

**Patentansprüche für den Vertragsstaat: AT**

1. Verfahren zur Herstellung eines Amides, dadurch gekennzeichnet, daß man eine Verbindung der Formel (II)

$$(X)_n—ArH \qquad (II)$$

in der X einen Rest aus der Gruppe von F, $CF_3$, $OCF_3$ und $SCF_3$ bedeutet, n gleich 1, 2 oder 3 ist, Ar den Phenylenrest oder einen mit einem Rest aus der Gruppe von Cl, Br, CN, $NO_2$, $NH_2$, CHO, COOH, COR, COOR, wobei R ein Alkylrest mit 1 bis 6 Kohlenstoffatomen ist, den Alkyl- und Alkoxyresten mit 1 bis 6 Kohlenstoffatomen, den Phenyl- und Phenoxyresten substituierten Phenylenrest bedeuten kann mit einer Verbindung der Formel (III)

$$OH—CH_2—NR_1—COR_2$$

in der $R_1$ und $R_2$ identisch oder verschieden sein können und Wasserstoff oder einen Alkylrest mit 1 bis 6 Kohlenstoffatomen oder einen Phenylrest bedeuten wobei $R_1$ und $R_2$ miteinander zu einem Ring verbunden sein können, in Gegenwart von Fluorwasserstoffsäure umsetzt.

2. Verfahren nach Anspruch 1, dadurch gekennzeichnet, daß die Fluorwasserstoffsäure wasserfrei ist.

3. Verfahren nach Anspruch 1, dadurch gekennzeichnet, daß Ar der Phenylenrest ist.

4. Verfahren nach einem der Ansprüche 1 bis 3, dadurch gekennzeichnet, daß n gleich 1 ist.

5. Verfahren nach einem der Ansprüche 1 bis 4, dadurch gekennzeichnet, daß in Formel (III) $R_1$ und $R_2$ gleichzeitig ein Wasserstoffatom bedeuten.

6. Verfahren nach einem der Ansprüche 1 bis 4, dadurch gekennzeichnet, daß in Formel (III) $R_1$ ein Wasserstoffatom und $R_2$ den Methylrest bedeutet.

7. Verfahren nach einem der Ansprüche 1 bis 6, dadurch gekennzeichnet, daß man eine derartige Menge Fluorwasserstoffsäure einsetzt, daß das Molverhältnis zwischen Fluorwasserstoffsäure und der Verbindung der Formel (II) zwischen 5 und 50 liegt.

8. Verfahren nach einem der Ansprüche 1 bis 7, dadurch gekennzeichnet, daß das Molverhältnis der Verbindung II zu der Verbindung III zwischen 0,5 und 2 liegt.

9. Verfahren nach einem der Ansprüche 1 bis 8, dadurch gekennzeichnet, daß man bei einer Temperatur zwischen 0° C und 100° C arbeitet.

**Claims for the contracting states: BE, CH, DE, FR, GB, IT, LI, LU, NL, SE**

1. N-Benzylfluorinated amides of the formula (I)

$$(X)_n-Ar-CH_2-NR_1-COR_2 \qquad (I)$$

in which:
X represents a radical chosen from among the group comprising F, $CF_3$, $OCF_3$ and $SCF_3$,
Ar represents the phenylene radical or a phenylene radical substituted by a radical chosen from among the group comprising Cl, Br, CN, $NO_2$, $NH_2$, CHO, COOH, COR and COOR, where R is an alkyl radical having from 1 to 6 carbon atoms, the alkyl and alkoxy radicals having from 1 to 6 carbon atoms, the phenyl radical and the phenoxy radical,
$R_1$ and $R_2$, which may be identical or different, each represent hydrogen or an alkyl radical having from 1 to 6 carbon atoms or a phenyl radical, it being possible for $R_1$ and $R_2$ to be linked to form a ring,
and n is 1, 2 or 3.

2. Amides according to Claim 1, characterised in that Ar represents the phenylene radical.

3. Amides according to any one of the preceding claims, characterised in that n = 1.

4. Amides according to any one of the preceding claims, characterised in that $R_1$ and $R_2$ simultaneously represent a hydrogen atom.

5. Amides according to Claims 1, 2, 3 and 4, characterised in that they have the formula:

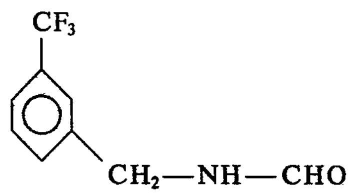
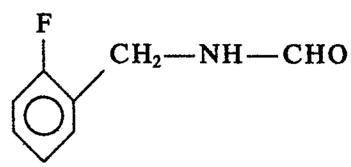
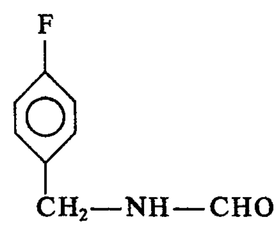
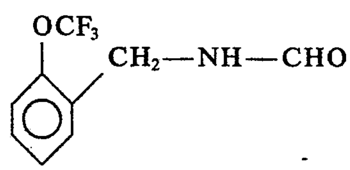
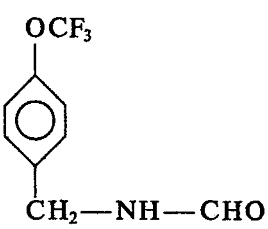
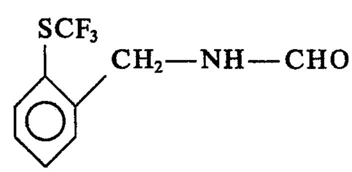
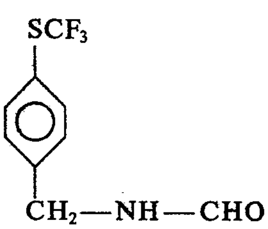

6. Amides according to any one of Claims 1 to 3, characterised in that $R_1$ represents a hydrogen atom and $R_2$ represents a methyl radical.

7. Amides according to Claim 1, 2, 3 and 6, characterised in that they have the formula:

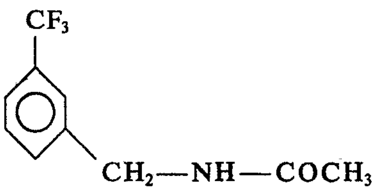 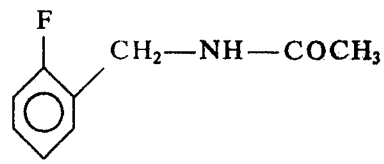 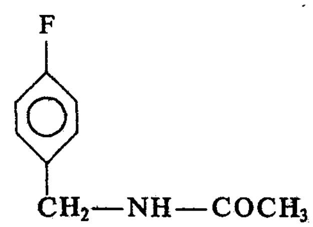

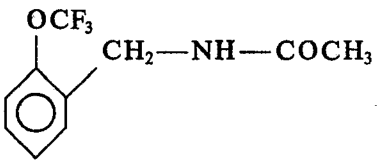 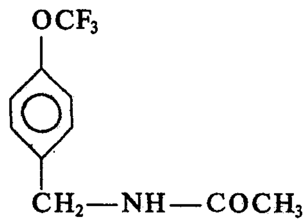 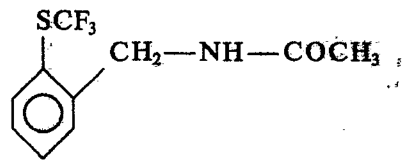

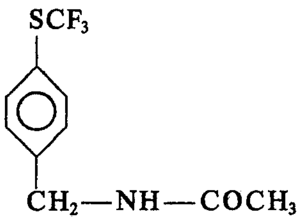

8. Process for the preparation of an amide according to any one of the preceding claims, characterised in that a compound of the formula (II)

$$(X)_n\text{—ArH} \qquad \text{(II)}$$

is reacted, in the presence of hydrofluoric acid, with a compound of the formula (III)

$$OH\text{—}CH_2\text{—}NR_1\text{—}COR_2 \qquad \text{(III)}$$

in which X, Ar, $R_1$ and $R_2$ have the same meaning as in Claim 1.

9. Process according to Claim 8, characterised in that the hydrofluoric acid is anhydrous hydrofluoric acid.

10. Process according to any one of Claims 8 and 9, characterised in that Ar represents the phenylene radical.

11. Process according to any one of Claims 8 to 10, characterised in that n = 1.

12. Process according to any one of Claims 8 to 11, characterised in that in the formula (III) $R_1$ and $R_2$ are, simultaneously, a hydrogen atom.

13. Process according to any one of Claims 8 to 11, characterised in that in the formula (III) $R_1$ represents a hydrogen atom and $R_2$ a methyl radical.

14. Process according to any one of Claims 8 to 13, characterised in that the amount of hydrofluoric acid used is such that the molar ratio of the hydrofluoric acid to the compound of the formula II is between 5 and 50.

15. Process according to any one of Claims 8 to 14, characterised in that the molar ratio of the compound II to the compound III is between 0.5 and 2.

16. Process according to any one of Claims 8 to 15, characterised in that it is carried out at a temperature of between 0° C and 100° C.

**Claims for the contracting state: AT**

1. Process for the preparation of an amide, characterised in that a compound of the formula (II)

$$(X)_n\text{—ArH} \qquad \text{(II)}$$

in which X represents a radical chosen from among the group comprising F, $CF_3$, $OCF_3$ and $SCF_3$, n is 1, 2 or 3 and Ar represents a phenylene radical or a phenylene radical substituted by a radical chosen from among the group comprising Cl, Br, CN, $NO_2$, $NH_2$, CHO, COOH, COR and COOR, where R is an alkyl radical having from 1 to 6 carbon atoms, the alkyl and alkoxy radicals having from 1 to 6 carbon atoms, the phenyl radical and the phenoxy radical, is reacted, in the presence of hydrofluoric acid, with a compound of the formula (III)

$OH-CH_2-NR_1-COR_2$

in which $R_1$ and $R_2$, which may be identical or different, represent hydrogen, an alkyl radical having from 1 to 6 carbon atoms or a phenyl radical, it being possible for $R_1$ and $R_2$ to be linked to form a ring.

2. Process according to Claim 1, characterised in that the hydrofluoric acid is anhydrous hydrofluoric acid.

3. Process according to Claim 1, characterised in that Ar represents the phenylene radical.

4. Process according to any one of Claims 1 to 3, characterised in that $n = 1$.

5. Process according to any one of Claims 1 to 4, characterised in that in the formula (III) $R_1$ and $R_2$ are simultaneously a hydrogen atom.

6. Process according to any one of Claims 1 to 4, characterised in that in the formula (III) $R_1$ represents a hydrogen atom and $R_2$ a methyl radical.

7. Process according to any one of Claims 1 to 6, characterised in that the amount of hydrofluoric acid employed is such that the molar ratio of the hydrofluoric acid to the compound of the formula II is between 5 and 50.

8. Process according to any one of Claims 1 to 7, characterised in that the molar ratio of the compound II to the compound III is between 0.5 and 2.

9. Process according to any one of Claims 1 to 8, characterised in that it is carried out at a temperature of between 0° C and 100° C.